(19) Europäisches Patentamt
European Patent Office
Office européen des brevets

(11) Numéro de publication : **0 415 802 B1**

# (12) FASCICULE DE BREVET EUROPEEN

(45) Date de publication du fascicule du brevet :
**10.03.93 Bulletin 93/10**

(51) Int. Cl.$^5$ : **A61K 7/13**

(21) Numéro de dépôt : **90401880.1**

(22) Date de dépôt : **29.06.90**

(54) **Procédé de teinture des fibres kératiniques à base de monohydroxyindole et d'hydroxyindole 5,6-disubstitué et composition.**

(30) Priorité : **03.07.89 FR 8908916**

(43) Date de publication de la demande :
**06.03.91 Bulletin 91/10**

(45) Mention de la délivrance du brevet :
**10.03.93 Bulletin 93/10**

(84) Etats contractants désignés :
**AT BE CH DE DK ES FR GB GR IT LI NL SE**

(56) Documents cités :
**EP-A- 0 271 186**
**FR-A- 2 625 435**
**FR-A- 2 636 235**

(73) Titulaire : **L'OREAL**
**14, Rue Royale**
**F-75008 Paris (FR)**

(72) Inventeur : **Grollier, Jean-François**
**16bis boulevard Morlkand**
**F-75004 Paris (FR)**
Inventeur : **Cotteret, Jean**
**15, Allée des Meuniers**
**F-78480 Verneuil-sur-Seine (FR)**

(74) Mandataire : **Casalonga, Axel et al**
**BUREAU D.A. CASALONGA - JOSSE**
**Morassistrasse 8**
**W-8000 München 5 (DE)**

EP 0 415 802 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à un procédé de teinture des fibres kératiniques et en particulier des cheveux, en mettant en oeuvre des monohydroxyindoles en association avec un ou plusieurs hydroxyindole(s) 5,6-disubstitué(s) et un agent oxydant constitué par l'acide periodique éventuellement salifié.

On a déjà proposé de teindre les cheveux ou la peau en utilisant les monohydroxyindoles comportant le groupement hydroxy en position 4,5,6 et/ou 7, en mettant en oeuvre différents oxydants dont en particulier l'acide periodique et ses sels solubles.

Un tel procédé est décrit notamment dans l'EP-A-0 271 186.

On a également proposé pour la teinture des cheveux vivants des compositions contenant le 5,6-dihydroxyindole à un pH de préférence acide, l'application de ces compositions étant suivie de l'application d'un oxydant constitué notamment par un periodate.

Un tel procédé est décrit plus particulièrement dans FR-A-1 166 172 mais il conduit à des nuances grises à noires ne couvrant pas la totalité des nuances souhaitées.

Ces procédés de l'état de la technique présentent cependant l'inconvénient de donner lieu à des colorations très sélectives, notamment lorsque les cheveux ont été sensibilisés par une permanente. On constate, en effet, dans ce cas, une différence importante dans la puissance tinctoriale entre les parties non sensibilisées et celles sensibilisées par la permanente.

Par ailleurs, ces procédés conduisent souvent à des problèmes de tâchage du cuir chevelu.

La demanderesse a découvert, de manière surprenante, qu'en associant un ou plusieurs monohydroxyindole(s) particulier(s) avec un ou plusieurs hydroxyindole(s) 5,6-disubstitué(s) défini(s) ci-après, en faisant suivre cette application par un rinçage, puis par l'application d'une composition contenant à titre d'agent oxydant de l'acide periodique ou un de ses sels solubles dans l'eau, on obtenait des colorations variées, en particulier sur des cheveux sensibilisés par des permanentes, particulièrement peu sélectives, résistantes aux lavages successifs, présentant un bon unisson et ne tâchant pas le cuir chevelu.

Un objet de l'invention est donc constitué par le procédé de teinture en plusieurs étapes, comportant l'application d'au moins un monohydroxyindole et d'hydroxyindole 5,6-disubstitué défini ci-après suivie, après rinçage, de celle d'acide periodique ou un sel de cet acide.

L'invention a enfin pour objet un dispositif à plusieurs compartiments ou kit de teinture, contenant les différentes compositions utilisables dans le procédé conforme à l'invention.

D'autres objets de l'invention apparaîtront à la lecture de la description et des exemples qui suivent.

Le procédé de teinture des fibres kératiniques, en particulier des cheveux humains, est essentiellement caractérisé par le fait que l'on applique, dans un premier temps, une composition (A) contenant dans un milieu approprié pour la teinture,

(i) au moins un monohydroxyindole de formule :

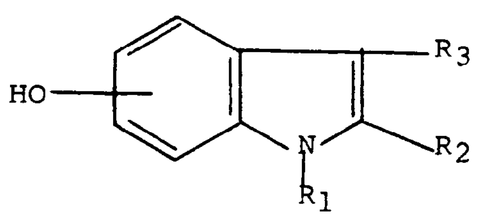

(I)

dans laquelle :

$R_1$ désigne hydrogène ou un radical alkyle, ayant 1 à 4 atomes de carbone;

$R_2$ et $R_3$ désignent, indépendamment l'un de l'autre, hydrogène; un groupement $NH_2$, OH, COOH, $CH_2COOH$ ou $CONH_2$; un radical alkyle ayant 1 à 4 atomes de carbone, éventuellement substitué par un ou plusieurs groupements OH ou $NH_2$; un radical alcoxy ayant 1 à 4 atomes de carbone; un groupement $CONHR_4$; un groupement CON $(R_5)(R_6)$ ou un groupement $CO_2R_4$; dans lesquels $R_4$, $R_5$, $R_6$ désignent un radical alkyle de 1 à 4 atomes de carbone; un atome d'halogène et

(ii) au moins un hydroxyindole 5,6-disubstitué de formule (II),

ou leur(s) mélange(s);

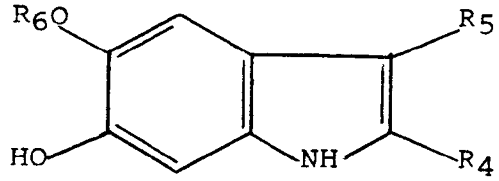

(II)

dans laquelle:

$R_6$ désigne hydrogène ou acétyle;

$R_4$ désigne hydrogène, méthyle, carboxy ou éthoxycarbonyle;

$R_6$ désigne hydrogène ou méthyle,

sous réserve que lorsque $R_6$ désigne acétyle, $R_4$ et $R_5$ représentent un atome d'hydrogène; et ses sels;

- que l'on procède dans un deuxième temps au rinçage des fibres kératiniques ainsi traitées, et
- que dans un troisième temps, on applique une composition (B) contenant, dans un milieu aqueux approprié pour la teinture, de l'acide periodique ou l'un de ses sels solubles dans le milieu aqueux.

Parmi les composés de formule (I), on peut citer :

- le 4-hydroxyindole
- le 5-hydroxyindole
- le 6-hydroxyindole
- le 7-hydroxyindole
- l'acide 5-hydroxyindole 2-carboxylique
- l'acide 5-hydroxyindole 3-acétique
- le 5-hydroxy 3-hydroxyéthylindole
- Le 5-hydroxy 2-hydroxyméthylindole.

Parmi ces composés, les composés préférés sont constitués par les 5-hydroxyindole, 4-hydroxyindole, 6-hydroxyindole et 7-hydroxyindole.

Parmi les composés de formule (II), on peut citer le 5,6-dihydroxyindole, le 5,6-dihydroxy 2-carboxy indole, le 5,6-dihydroxy 2-éthoxycarbonylindole, le 5-acétoxy 6-hydroxyindole, le 2,3-diméthyl 5,6-dihydroxy indole, le 2-méthyl 5,6-dihydroxyindole. Le 5,6-dihydroxyindole est particulièrement préféré.

Dans la composition (A), le(s) composé(s) de formule (II) et le(s) monohydroxyindole(s) de formule (I) sont présents dans des concentrations suffisantes pour obtenir, après application de la composition à base d'acide periodique éventuellement salifié, une coloration sur les fibres kératiniques et en particulier sur les cheveux. Les concentrations de chacun d'eux sont comprises de préférence entre 0,01 et 5% en poids, et en particulier entre 0,05 et 3% en poids, par rapport au poids total de la composition.

Les sels d'acide periodique utilisables sont les sels solubles dans l'eau, tels que les sels de lithium, sodium, potassium, rhubidium, césium, magnésium, calcium, strontium, manganèse, fer, cuivre, zinc, aluminium, les sels de sodium et de potassium étant particulièrement préférés.

L'acide periodique éventuellement salifié est présent dans des concentrations suffisantes pour oxyder les composés de formule (I) et de formule (II). Ces proportions sont comprises de préférence entre 0,004 et 0,07 mole et en particulierentre 0,01 mole et 0,04 mole pour 100g de composition d'acide périodique éventuellement salifié.

Conformément à l'invention, la composition (A) est maintenue au contact des cheveux pendant un temps de pose compris entre 1 minute et 60 minutes, de préférence entre 10 minutes et 40 minutes, avant d'être soumise au rinçage. De même la composition (B) contenant l'acide périodique ou ses sels est maintenue au contact des cheveux pendant un temps de pose compris entre 1 minute et 60 minutes et de préférence entre 10 minutes et 40 minutes avant que les cheveux ne soient rincés et éventuellement lavés.

Les temps de pose des compositions (A) et (B) peuvent être égaux ou différents.

Les compositions utilisées conformément à l'invention, peuvent se présenter sous des formes diverses, habituellement utilisées dans le domaine de la teinture des fibres kératiniques, et notamment des cheveux, et en particulier sous forme de liquides plus ou moins épaissis, de crèmes, de mousses, d'émulsions de gels et d'huiles.

Les compositions (A) et (B) mises en oeuvre, conformément à l'invention, comportent généralement un milieu aqueux constitué par de l'eau ou un mélange eau-solvant(s). La composition (A) peut être aussi constituée par un milieu solvant anhydre que l'on mélange au moment de l'emploi avec un milieu aqueux ou que l'on applique directement sur les fibres humides.

On appelle "milieu anhydre" un milieu contenant moins de 1% d'eau.

Les solvants utilisables, conformément à l'invention, sont choisis parmi les alcools aliphatiques, tels que

l'alcool éthylique, l'alcool propylique, isopropylique ou l'alcool tertiobutylique; les polyols, tels que l'éthylèneglycol, le diéthylèneglycol, le propylèneglycol, le glycérol ou le dipropylèneglycol; les éthers monométhylique, monoéthylique ou monobutylique d'éthylèneglycol, de diéthylèneglycol, de propylèneglycol ou de dipropylèneglycol; l'acétate du monométhyléther d'éthylèneglycol, le lactate de méthyle. La proportion de solvants peut varier de préférence entre 1 et 50% en poids par rapport au poids total de chaque composition.

Ces compositions peuvent contenir des agents tensio-actifs anioniques, cationiques, non ioniques ou amphotères ou leurs mélanges dans une teneur totale préférentielle comprise entre 0,5 et 20% en poids par rapport au poids total de la composition.

Elles peuvent également contenir des agents épaississants, tels que l'alginate de sodium, la gomme arabique, la gomme de guar et ses dérivés, les dérivés de cellulose, la gomme de xanthane, les scléroglucanes, les polymères dérivés de l'acide acrylique, la bentonite.

Les proportions en agents épaississants peuvent varier entre 0,1 et 5% par rapport au poids total de chaque composition.

Lorsque la composition (A) est aqueuse, son pH est compris de préférence entre 3 et 10 et en particulier entre 4 et 7.

Le pH de la composition (B) est compris de préférence entre 2 et 10 et en particulier entre 2,5 et 5.

Les compositions mises en oeuvre dans le procédé conforme à l'invention peuvent également contenir d'autres adjuvants habituellement utilisés dans les teintures des fibres kératiniques, tels que des agents séquestrants, des agents antioxydants ou réducteurs, des agents filmogènes, des agents nacrants, des agents dispersants, des agents de traitement, des parfums, des tampons, des électrolytes, des agents de pénétration) etc...

Si les compositions sont utilisées sous forme de mousse, elles peuvent être conditionnées sous pression dans un dispositif aérosol en présence d'un agent propulseur et d'au moins un agent générateur de mousse.

Le procédé conforme à l'invention peut être mis en oeuvre pour la teinture de tous types de cheveux, mais également pour la teinture des fourrures ou de la laine.

Un autre objet de l'invention est constitué par une composition à composants multiples séparés, comprenant un premier composant (A) constitué par la composition (A) définie ci-dessus et contenant dans un milieu acceptable, au moins un monohydroxyindole tel que défini par la formule (I) et au moins un hydroxyindole 5,6-disubstitué de formule (II) et un composant (B) constitué par la composition (B) contenant dans un milieu acceptable, l'acide periodique ou ses sels hydrosolubles.

L'invention a également pour objet un kit de teinture ou dispositif à plusieurs compartiments, comportant un premier compartiment contenant la composition (A) définie ci-dessus et un second compartiment contenant la composition (B) également définie ci-dessus. Ce dispositif peut éventuellement contenir un troisième compartiment contenant un milieu aqueux destiné à être mélangé au contenu du premier compartiment lorsque le milieu utilisé pour la composition (A) est anhydre.

Les exemples qui suivent sont destinés à illustrer l'invention sans pour autant présenter un caractère limitatif.

EXEMPLE 1

Composition (A) :

| | |
|---|---|
| - 5,6-dihydroxyindole | 0,4 g |
| - 5-hydroxyindole | 0,6 g |
| - Alcool éthylique | 10,0 g |
| - Gomme de guar vendue sous la dénomination JAGUAR HP60 par la Société CELANESE | 1,0 g |
| - Alkyléther de glycoside vendu à la concentration de 60% MA sous la dénomination TRITON CG 110 par la Société SEPPIC | 5,0 g MA |
| - Conservateurs qs | |
| - pH ajusté | entre 6,5 et 6,9 |
| - Eau déminéralisée | qsp 100,0 g |

Composition (B) :

| | |
|---|---|
| - Métaperiodate de sodium | 5,0 g |
| - HCl qs pH = 3 | |
| - Eau déminéralisée | qsp 100,0 g |

On applique la composition (A) sur des cheveux gris à 90% de blancs. Après 15 minutes de pose, les cheveux sont rincés. On applique alors la composition (B) pendant 15 minutes. On rince à nouveau et on sèche. Les cheveux sont alors colorés dans une nuance blond foncé.

Cette nuance est peu sélective à la permanente.

EXEMPLE 2

Les 0,6 g de 5-hydroxyindole de la composition (A) de l'exemple 1 sont remplacés par 0,6 g de 6-hydroxyindole.

On obtient en final une nuance châtain clair peu sélective à la permanente.

EXEMPLE 3

Les 0,6 g de 5-hydroxyindole de la composition (A) de l'exemple 1 sont remplacés par 0,6 g de 4-hydroxyindole. On obtient en final une nuance châtain cendré peu sélective à la permanente.

EXEMPLE 4

Les 0,6 g de 5-hydroxyindole de la composiiton (A) de l'exemple 1 sont remplacés par 0,6 g de 7-hydroxyindole. On obtient en final une nuance châtain clair acajou violine peu sélective à la permanente.

EXEMPLE 5

Composition (A) :

| | | |
|---|---|---|
| - 5,6-dihydroxyindole | | 0,2 g |
| - 6-hydroxyindole | | 0,4 g |
| - Alcool éthylique | | 10,0 g |
| - Gomme de Guar vendue sous la dénomination JAGUAR HP 60 par la Société CELANESE | | 1,0 g |
| - Alkyléther de glycoside vendu à la concentration de 60% de MA sous la dénomination TRITON CG 110 par la Société SEPPIC | | 5,0 g MA |
| - pH ajusté | | entre 6,5 et 6,9 |
| - Conservateurs qs | | |
| - Eau | qsp | 100,0 g |

On applique la composition (A) pendant 15 mn sur des cheveux gris à 90% de blancs. Les cheveux sont rincés. On applique alors la composition (B) décrite à l'exemple 1 pendant 15 mn. On rince et on sèche. Les cheveux sont colorés en une nuance blond cendré peu sélective à la permanente.

EXEMPLE 6

On reproduit l'exemple 1, avec 0,1 g de 5,6-dihydroxyindole au lieu de 0,4 g et en remplaçant les 0,6 g de 5-hydroxyindole par 0,25 g de 4-hydroxyindole ; la nuance obtenue est gris moyen uniforme ; elle est peu sélective à la permanente.

EXEMPLE 7

On reproduit l'exemple 1, avec 0,1 g de 5,6-dihydroxyindole au lieu de 0,4 g et en remplaçant les 0,6 g de 5-hydroxyindole par 0,25 g de 7-hydroxyindole ; la nuance obtenue est blond foncé acajou ; elle est peu sélective à la permanente.

On a constaté pour les différents exemples mentionnés ci-dessus une diminution importante de la sélectivité grâce à l'association, cette diminution importante de sélectivité étant constatée en déterminant la couleur dans le système de MUNSELL, sur des cheveux gris naturels, d'une part, et sur des cheveux gris permanentés, d'autre part.

On constate que la différence de couleur entre ces deux types de cheveux déterminée selon la formule de NICKERSON : $\Delta E = 0{,}4\ C_o\ dH + 6\ dV + 3dC$ définie dans : Journal of the Optical Society of America, Volume 34, N°9, Septembre 1944, Pages 550 à 570, est sensiblement moins importante dans le cas du procédé de l'invention.

Dans cette équation, $\Delta E$ représente la variation de couleur totale, dH, dV et dC représentent les variations, en valeur absolue, des paramètres H, V et C de MUNSELL, $C_o$ étant la chromaticité de la couleur initiale.

EXEMPLE 8

Composition (A) :

| | | |
|---|---|---|
| - 5-hydroxyindole | | 0,5 g |
| - 5,6-dihydroxyindole | | 0,3 g |
| - Bromhydrate de 2,3-diméthyl 5,6-dihydroxyindole | | 0,3 g |
| - Alcool éthylique | | 10,0 g |
| - Hydroxyéthylcellulose vendue sous la dénomination NATROSOL 250 HHR par la Société AQUALON | | 0,5 g |
| - Lauryléthersulfate de sodium | | 0,1 g MA |
| - Triéthanolamine qs pH 6,5 | | |
| - Eau | qsp | 100,0 g |

On applique la composition (A) pendant 15 minutes sur des cheveux gris à 90% de blancs. Les cheveux sont rincés.

On applique alors la composition (B) décrite à l'exemple 1 pendant 15 minutes. Après rinçage, lavage au shampooing et séchage, les cheveux sont colorés en châtain foncé naturel acajou.

EXEMPLE 9

Composition (A) :

| | | |
|---|---|---|
| - 6-hydroxyindole | | 0,5 g |
| - 5,6-dihydroxy 2-éthoxycarbonylindole | | 0,5 g |
| - Alcool éthylique | | 10,0 g |
| - Hydroxyéthylcellulose vendue sous la dénomination NATROSOL 250 HHR par la Société AQUALON | | 0,5 g |
| - Lauryléthersulfate de sodium | | 0,1 g MA |
| - Triéthanolamine qs pH 6,5 | | |
| - Eau | qsp | 100,0 g |

On applique la composition (A) pendant 30 minutes sur des cheveux gris à 90% de blancs. Les cheveux sont rincés.

On applique alors la composition (B) décrite à l'exemple 1 pendant 15 minutes. Après rinçage, lavage au shampooing et séchage, les cheveux sont colorés en châtain naturel doré.

EXEMPLE 10

Composition (A) :

| | | |
|---|---|---|
| - 7-hydroxyindole | | 0,2 g |
| - 5,6-dihydroxyindole | | 0,1 g |
| - Bromhydrate de 2-méthyl 5,6-dihydroxyindole | | 0,5 g |
| - Alcool éthylique | | 10,0 g |
| - Hydroxyéthylcellulose vendue sous la dénomination NATROSOL 250 HHR par la Société AQUALON | | 0,5 g |
| - Lauryléthersulfate de sodium | | 0,1 g MA |
| - Triéthanolamine qs pH 6,5 | | |
| - Eau | qsp | 100,0 g |

On applique la composition (A) pendant 15 minutes sur des cheveux gris permanentés. Les cheveux sont rincés.

On applique alors la composition (B) décrite à l'exemple 1 pendant 30 minutes. Après rinçage, lavage au shampooing et séchage, les cheveux sont colorés en châtain foncé acajou.

EXEMPLE 11

Composition (A) :

| | | |
|---|---|---|
| - 6-hydroxyindole | | 0,2 g |
| - 5,6-dihydroxyindole | | 0,4 g |
| - 5,6-dihydroxy 2-carboxyindole | | 0,4 g |
| - Hydroxyéthylcellulose vendue sous la dénomination NATROSOL 250 HHR par la Société AQUALON | | 1,0 g |
| - Alcool éthylique | | 10,0 g |
| - Lauryléther sulfate de sodium | | 0,2 g |
| - Triéthanolamine qs pH 6,5 | | |
| - Eau | qsp | 100,0 g |

Composition (B) :

| | |
|---|---|
| - Métaperiodate de sodium | 2,5 g |
| - HCl qs pH 3 | |
| - Eau | qsp 100,0 g |

On applique pendant 15 minutes la composition (A) sur des cheveux gris permanentés. Les cheveux sont rincés.

On applique alors la composition (B) pendant 15 minutes. Après rinçage, lavage au shampooing et séchage, les cheveux sont teints en brun.

EXEMPLE 12

Composition (A) :

| | |
|---|---|
| - 5-hydroxyindole | 0,3 g |
| - 5,6-dihydroxyindole | 0,1 g |
| - 5-acétoxy 6-hydroxyindole | 0,3 g |
| - Hydroxyéthylcellulose vendue sous la dénomination NATROSOL 250 HHR par la Société AQUALON | 1,0 g |
| - Alcool éthylique | 10,0 g |
| - Lauryléther sulfate de sodium | 0,2 g |
| - Triéthanolamine qs pH 6,5 | |
| - Eau | qsp 100,0 g |

On applique la composition (A) pendant 15 minutes sur des cheveux gris à 90% de blancs. Les cheveux sont rincés.

On applique alors la composition (B) décrite à l'exemple 11 pendant 15 minutes. Après rinçage, lavage au shampooing et séchage, les cheveux sont teints en châtain clair irisé.

EXEMPLE 13

Composition (A) :

| | | | |
|---|---|---|---|
| - 5-hydroxyindole | | 0,05 | g |
| - 5,6-dihydroxyindole | | 3,5 | g |
| - Hydroxyéthylcellulose vendue sous la dénomination NATROSOL 250 HHR par la Société AQUALON | | 1,0 | g |
| - Alcool éthylique | | 10,0 | g |
| - Lauryléther sulfate de sodium | | 0,2 | g MA |
| - Triéthanolamine qs pH 6,5 | | | |
| - Eau | qsp | 100,0 | g |

On applique la composition (A) pendant 15 minutes sur des cheveux gris à 90% de blancs. Les cheveux sont rincés.

On applique alors la composition (B) décrite à l'exemple 11 pendant 15 minutes. Après rinçage, lavage au shampooing et séchage, les cheveux sont teints en brun.

**Revendications**

**1.** Procédé de teinture des fibres kératiniques, caractérisé par le fait que l'on applique sur ces fibres,

dans un premier temps, une composition (A) contenant, dans un milieu approprié pour la teinture :

(i) au moins un monohydroxyindole répondant à la formule (I) :

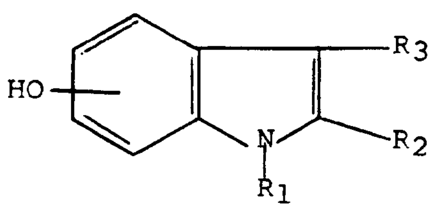

(I)

dans laquelle :

$R_1$ désigne hydrogène ou un radical alkyle en $C_1$-$C_4$;

$R_2$ et $R_3$ désignent, indépendamment l'un de l'autre, hydrogène, un groupement $NH_2$, OH, COOH, $CH_2COOH$ ou $CONH_2$; un radical alkyle en $C_1$-$C_4$, éventuellement substitué par un ou plusieurs groupements OH ou $NH_2$; un radical alcoxy ayant 1 à 4 atomes de carbone; un groupement $CONHR_4$ ou un groupement CON ($R_5$) ($R_6$) ou un groupement $CO_2R_4$, dans lesquels $R_4$, $R_5$, $R_6$ désignent un radical alkyle de 1 à 4 atomes de carbone; un atome d'halogène, et

(ii) au moins un hydroxyindole 5,6-disubstitué de formule (II),

ou leur(s) mélange(s);

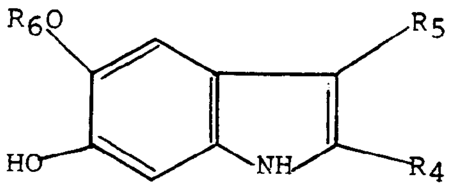

(II)

dans laquelle :

$R_6$ désigne hydrogène ou acétyle;

$R_4$ désigne hydrogène, méthyle, carboxy ou éthoxycarbonyle;

$R_5$ désigne hydrogène ou méthyle,

sous réserve que lorsque $R_6$ désigne acétyle, $R_4$ et $R_5$ représentent un atome d'hydrogène; et ses sels;

- que l'on procède, dans un deuxième temps, au rinçage des fibres traitées avec la composition appliquée dans le premier temps; et
- que dans un troisième temps, on applique une composition (B) contenant dans un milieu aqueux approprié pour la teinture, de l'acide periodique ou l'un de ses sels soluble dans ce milieu.

2. Procédé selon la revendication 1, caractérisé par le fait que le monohydroxyindole est choisi parmi le 4-hydroxyindole, le 5-hydroxyindole, le 6-hydroxyindole, le 7-hydroxyindole, l'acide 5-hydroxyindole 2-carboxylique, l'acide 5-hydroxyindole 3-acétique, le 5-hydroxy 3-hydroxyéthylindole, le 5-hydroxy 2-hydroxyméthylindole.

3. Procédé selon la revendication 1 ou 2, caractérisé par le fait que l'hydroxyindole 5,6-disubstitué de formule (II) est le 5,6-dihydroxyindole.

4. Procédé selon la revendication 1 ou 2, caractérisé par le fait que l'hydroxyindole 5,6-disubstitué est choisi parmi le 5,6-dihydroxy 2-carboxy indole, le 5,6-dihydroxy 2-éthoxycarbonylindole, le 5-acétoxy 6-hydroxyindole, le 2,3-diméthyl 5,6-dihydroxyindole, le 2-méthyl 5,6-dihydroxyindole.

5. Procédé de teinture des fibres kératiniques caractérisé par le fait qu'on applique sur ces fibres,
   - dans un premier temps, une composition (A) contenant dans un milieu approprié pour la teinture :
     (i) au moins un monohydroxyindole choisi parmi le 4-hydroxyindole, le 5-hydroxyindole, le 6-hydroxyindole et le 7-hydroxyindole,
     (ii) au moins du 5,6-dihydroxyindole ;
   - que l'on rince dans un deuxième temps, les fibres ainsi traitées.
   - que l'on applique, dans un troisième temps une composition (B) contenant dans un milieu aqueux approprié pour la teinture, de l'acide periodique ou l'un de ses sels soluble dans ce milieu.

6. Procédé selon l'une quelconque des revendications 1 à 5, caractérisé par le fait que les sels d'acide periodique sont choisis parmi les sels de lithium, de sodium, de potassium, de rubidium, de césium, de magnésium, de calcium, de strontium, de manganèse, de fer, de cuivre, de zinc et d'aluminium.

7. Procédé selon l'une quelconque des revendications 1 à 6, caractérisé par le fait que le monohydroxyindole de formule (I) et l'hydroxyindole 5,6-disubstitué de formule (II) sont présents chacun dans des concentrations respectives comprises entre 0,01 et 5% en poids, de préférence entre 0,05 et 3% en poids, par rapport au poids total de la composition.

8. Procédé selon l'une quelconque des revendications 1 à 6, caractérisé par le fait que l'acide periodique ou ses sels sont présents dans des proportions comprises entre 0,004 et 0,07 mole et de préférence entre 0,01 et 0,04 mole pour 100 g de composition.

9. Procédé selon l'une quelconque des revendications 1 à 8, caractérisé par le fait que l'on applique la composition (A) pendant un temps de pose de 1 minute à 60 minutes avant de procéder au rinçage et la composition (B) avec un temps de pose compris entre 1 minute et 60 minutes, les temps de pose des compositions (A) et (B) pouvant être identiques ou différents.

10. Procédé selon l'une quelconque des revendications 1 à 9, caractérisé par le fait que les compositions mises en oeuvre comportent un milieu aqueux constitué par de l'eau ou un mélange eau-solvant(s).

**11.** Procédé selon l'une quelconque des revendications 1 à 9, caractérisé par le fait que le milieu de la composition (A) est constitué par un milieu solvant anhydre.

**12.** Procédé selon l'une quelconque des revendications 10 ou 11, caractérisé par le fait que les solvants sont choisis parmi les alcools aliphatiques, les polyols, les éthers monométhylique, monoéthylique ou monobutylique de l'éthylèneglycol, du diéthylèneglycol, du propylèneglycol ou du dipropylèneglycol, l'acétate du monométhyléther de l'éthylèneglycol, le lactate de méthyle.

**13.** Procédé selon l'une quelconque des revendications 1 à 10 et 12, caractérisé par le fait que la composition (A) est aqueuse et a un pH compris entre 3 et 10 et de préférence compris entre 4 et 7.

**14.** Procédé selon l'une quelconque des revendications 1 à 10 et 12, caractérisé par le fait que la composition (B) a un pH compris entre 2 et 10 et de préférence entre 2,5 et 5.

**15.** Procédé selon l'une quelconque des revendications 1 à 14, caractérisé par le fait que le milieu des compositions (A) et (B) est épaissi avec des agents épaississants présents dans des proportions comprises entre 0,1 et 5% en poids par rapport au poids total de la composition.

**16.** Procédé selon l'une quelconque des revendications 1 à 15, caractérisé par le fait que le milieu des compositions (A) et (B) contient également les adjuvants habituellement utilisés pour la teinture des fibres kératiniques choisis parmi les tensio-actifs, les agents séquestrants, les agents antioxydants ou réducteurs, les agents filmogènes, les agents nacrants, les agents dispersants, les agents de traitement, les agents de pénétration, des parfums, les tampons et les électrolytes.

**17.** Composition à plusieurs composants séparés, caractérisée par le fait qu'elle comprend un premier composant constitué par une composition (A) contenant, dans un milieu cosmétiquement acceptable, au moins un monohydroxyindole répondant à la formule (I) :

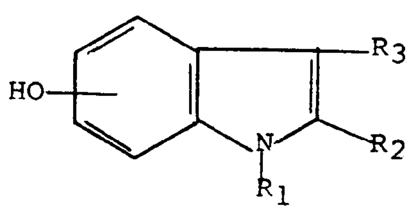

(I)

dans laquelle :

$R_1$ désigne hydrogène ou un radical alkyle, ayant 1 à 4 atomes de carbone;

$R_2$ et $R_3$ désignent, indépendamment l'un de l'autre, hydrogène; un groupement $NH_2$, OH, COOH, $CH_2COOH$, $CONH_2$; un radical alkyle de 1 à 4 atomes de carbone, éventuellement substitué par un ou plusieurs groupements OH ou $NH_2$; un radical alcoxy de 1 à 4 atomes de carbone; un groupement $CONHR_4$ ou un groupement CON ($R_5$) ($R_6$) ou un groupement $CO_2R_4$, dans lesquels $R_4$, $R_5$, $R_6$ désignent un radical alkyle de 1 à 4 atomes de carbone; un atome d'halogène et au moins un hydroxy indole 5,6-disubstitué de formule (II) :

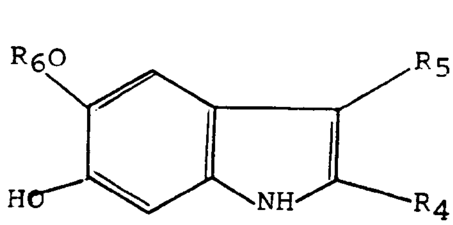

(II)

dans laquelle :

$R_6$ désigne hydrogène ou acétyle;

$R_4$ désigne hydrogène, méthyle, carboxy ou éthoxycarbonyle;

$R_5$ désigne hydrogène ou méthyle,

sous réserve que lorsque $R_6$ désigne acétyle, $R_4$ et $R_5$ représentent un atome d'hydrogène; et ses sels;

et un deuxième composant constitué par une composition (B) contenant dans un milieu cosmétiquement acceptable l'acide periodique ou ses sels hydrosolubles.

**18.** Dispositif à plusieurs compartiments ou kits de teinture, caractérisé par le fait qu'il comprend un premier compartiment contenant la composition (A) telle que définie dans l'une quelconque des revendications 1 à 13, 15 et 16 et un second compartiment contenant la composition (B) telle que définie dans l'une quelconque des revendications 1 à 10, 12, 14, 15, et 16.

## Patentansprüche

**1.** Verfahren zum Färben von keratinischen Fasern, dadurch gekennzeichnet, daß man auf die Fasern in einer ersten Stufe eine Zusammensetzung (A) aufbringt, die in einem zur Färbung geeigneten Milieu enthält:

(i) mindestens ein Monohydroxyindol der Formel:

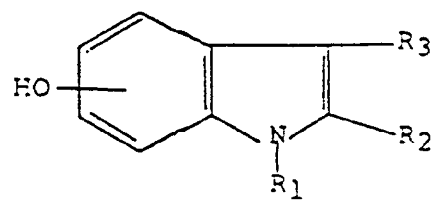

(I)

worin bedeuten:

$R_1$ Wasserstoff oder ein $C_1$ bis $C_4$-Alkylradikal;

$R_2$ und $R_3$ unabhängig voneinander Wasserstoff, eine $NH_2$-, OH-, COOH-, $CH_2COOH$ oder $CONH_2$-Gruppe; ein $C_1$ bis $C_4$-Alkylradikal, das gegebenenfalls durch eine oder mehrere OH- oder $NH_2$-Gruppen substituiert ist; ein Alkoxyradikal mit 1 bis 4 Kohlenstoffatomen; eine Gruppe $CONHR_4$; eine Gruppe CON $(R_6)(R_6)$ oder eine Gruppe $CO_2R_4$, worin $R_4$, $R_6$, und $R_6$ ein Alkylradikal mit 1 bis 4 Kohlenstoffatomen bedeuten; ein Halogenatom und

(ii) mindestens ein 5,6-disubstituiertes Hydroxyindol der Formel (II), oder ihre Mischungen;

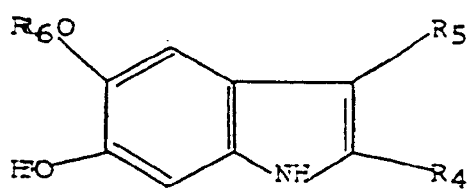

(II)

worin bedeuten:

$R_6$ Wasserstoff oder Acetyl;

$R_4$ Wasserstoff, Methyl, Carboxy oder Ethoxycarbonyl;

$R_5$ Wasserstoff oder Methyl, mit der Maßgabe, daß, wenn $R_6$ Acetyl bedeutet, $R_4$ und $R_5$ ein Wasserstoffatom bedeuten; und ihre Salze;

- und in einer zweiten Stufe die mit der Zusammensetzung der ersten Stufe behandelten Haare spült; und
- in einer dritten Stufe eine Zusammensetzung (B) in einem zur Färbung geeigneten wässerigen Milieu appliziert, die Periodsäure oder eines ihrer in diesem Milieu löslichen Salze enthält.

**2.** Verfahren nach Anspruch 1, dadurch gekennzeichnet daß, das Monohydroxyindol ausgewählt ist unter 4-Hydroxyindol, 5-Hydroxyindol, 6-Hydroxyindol, 7-Hydroxyindol, 5-Hydroxyindol-2-carbonsäure, 5-Hydroxyindol-3-essigsäure, 5-Hydroxy-3-hydroxyethylindol, 5-Hydroxy-2-hydroxymethylindol.

**3.** Verfahren gemäß Anspruch 1 oder 2, dadurch gekennzeichnet, daß das 5,6-disubstituierte Hydroxyindol der Formel (II) 5,6-Dihydroxyindol ist.

4. Verfahren gemäß Anspruch 1 oder 2, dadurch gekennzeichnet, daß das 5,6-disubstituierte Hydroxyindol ausgewählt ist unter 5,6-Dihydroxy-2-carboxy-indol, 5,6-Dihydroxy-2-hydroxycarbonyl-indol, 5-Acetoxy-6-hydroxy-indol, 2,3-Dimethyl-5,6-dihydroxy-indol, 2-Methyl-5,6-dihydroxyindol.

5. Verfahren zum Färben von keratinischen Fasern, dadurch gekennzeichnet, daß man auf die Fasern
   - in einer ersten Stufe eine Zusammensetzung (A) aufbringt, die in einem zur Färbung geeigneten Milieu enthält:
     (i) mindestens ein Monohydroxyindol ausgewählt aus 4-Hydroxyindol, 5-Hydroxyindol, 6-Hydroxyindol und 7-Hydroxyindol,
     (ii) mindestens ein 5,6-Dihydroxy-indol;
   - daß man in einer zweiten Stufe die so behandelten Fasern spült,
   - daß man in einer dritten Stufe eine Zusammensetzung (B) appliziert, die ein in einem wässerigen zur Färbung geeigneten Milieu Periodsäure oder eines seiner in diesem Milieu löslichen Salzes enthält.

6. Verfahren nach einem der Ansprüche 1 bis 5, dadurch gekennzeichnet, daß die Salze der Periodsäure ausgewählt sind unter den Salzen von Lithium, Natrium, Kalium, Rhubidium, Cäsium, Magnesium, Calcium, Strontium, Mangan, Eisen, Kupfer, Zink und Aluminium.

7. Verfahren gemäß einem der Ansprüche 1 bis 6, dadurch gekennzeichnet, daß das Monohydroxyindol der Formel (I) und das 5,6-disubstituierte Hydroxyindol der Formel (II) jeweils in Konzentrationen zwischen 0.01 und 5 Gew.-%, vorzugsweise zwischen 0.05 und 3 % Gew.-%, bezogen auf das Gesamtgewicht der Zusammensetzung, vorhanden sind.

8. Verfahren gemäß einem der Ansprüche 1 bis 6, dadurch gekennzeichnet, daß die Periodsäure oder eines seiner Salze in Anteilen zwischen 0.004 und 0.07 Mol, und vorzugsweise zwischen 0.01 und 0.04 Mol auf 100 g der Zusammensetzung vorhanden sind.

9. Verfahren nach einem der Ansprüche 1 bis 8, dadurch gekennzeichnet, daß man die Zusammensetzung (A) während einer Einwirkungszeit von 1 Minute bis 60 Minuten appliziert, bevor man spült, und die Zusammensetzung (B) mit einer Einwirkungszeit zwischen 1 Minute und 60 Minuten, wobei die Einwirkungszeiten der Zusammensetzung (A) und (B) gleich oder verschieden sein können.

10. Verfahren gemäß einem der Ansprüche 1 bis 9, dadurch gekennzeichnet, daß das wässerige Milieu der Zusammensetzungen sich aus Wasser oder einer Wasser-Lösungsmittel-Mischung zusammensetzt.

11. Verfahren nach einem der Ansprüche 1 bis 9, dadurch gekennzeichnet, daß das Milieu der Zusammensetzung (A) ein wasserfreies Lösungsmittelmilieu ist.

12. Verfahren gemäß einem der Ansprüche 10 oder 11, dadurch gekennzeichnet, daß die Lösungsmittel ausgewählt sind unter den aliphatischen Alkoholen, den Polyolen, den Monomethyl-, Monoethyl-, oder Monobutyl-Ethern von Ethylenglykol, Diethylenglykol, Propylenglykol oder Dipropylenglykol, Monomethyletheracetat von Ethylenglykol, Methyllactat.

13. Verfahren nach einem der Ansprüche 1 bis 10 und 12, dadurch gekennzeichnet, daß die Zusammensetzung (A) eine wässerige Zusammensetzung ist, und einen pH-Wert zwischen 3 und 10, und vorzugsweise zwischen 4 und 7, besitzt.

14. Verfahren gemäß einem der Ansprüche 1 bis 10 und 12, dadurch gekennzeichnet, daß die Zusammensetzung (B) einen pH-Wert zwischen 2 und 10, und vorzugsweise zwischen 2.5 und 5, besitzt.

15. Verfahren gemäß einem der Ansprüche 1 bis 14, dadurch gekennzeichnet, daß das Milieu der Zusammensetzung (A) und (B) mittels Verdickungsmitteln verdickt ist, die in Anteilen zwischen 0.1 und 5 Gew.-%, bezogen auf das Gesamtgewicht der Zusammensetzung, vorhanden sind.

16. Verfahren nach einem der Ansprüche 1 bis 5, dadurch gekennzeichnet, daß das Milieu der Zusammensetzung (A) und (B) außerdem übliche, zum Färben keratinischer Fasern verwendete Additive enthält, ausgewählt unter den oberflächenaktiven Stoffen, Sequestrierungsmitteln, Antioxidantien oder Reduktionsmitteln, filmbildenden Mitteln, Glanzmitteln, Dispersionsmitteln, Formgebungsmitteln, Penetrationsmitteln, Parfüms, Puffern und Elektrolyten.

**17.** Zusammensetzung aus mehreren getrennten Komponenten, dadurch gekennzeichnet, daß eine erste Komponente durch eine Zusammensetzung (A) gebildet wird, die in einem kosmetisch annehmbaren Milieu mindestens ein Monohydroxyindol der Formel (I) enthält:

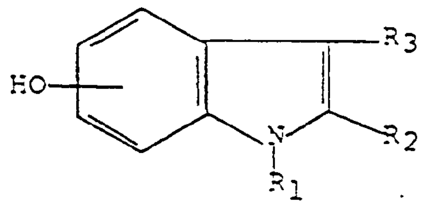

(I)

worin bedeuten:

$R_1$ ein Wasserstoffatom oder ein Alkylradikal mit 1 bis 4 Kohlenstoffatomen;

$R_2$ und $R_3$ unabhängig voneinander Wasserstoff, eine $NH_2$-, OH-, COOH-, $CH_2COOH$-, $CONH_2$-Gruppe; ein Alkylradikal mit 1 bis 4 Kohlenstoffatomen, das gegebenenfalls durch eine oder mehrere OH- oder $NH_2$-Gruppen substituiert ist; ein Alkoxyradikal mit 1 bis 4 Kohlenstoffatomen; eine $CONHR_4$-Gruppe oder eine $CON(R_5)(R_6)$-Gruppe oder eine Gruppe $CO_2R_4$, worin $R_4$, $R_6$ und $R_6$ ein Alkylradikal mit 1 bis 4 Kohlenstoffatomen bedeutet; ein Halogenatom,

und mindestens ein 5,6-disubstituiertes Indol der Formel (II):

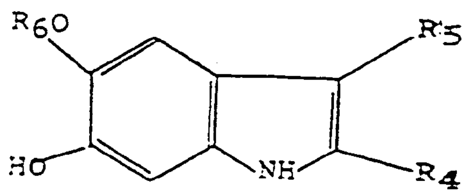

(II)

worin bedeuten:

$R_6$ Wasserstoff oder Acetyl;

$R_4$ Wasserstoff, Methyl, Carboxy oder Ethoxycarbonyl;

$R_5$ Wasserstoff oder Methyl, mit der Maßgabe, daß, wenn $R_6$ Acetyl bedeutet, $R_4$ und $R_5$ ein Wasserstoffatom bedeuten; und ihren Salze;

und eine zweite Komponente, die aus der Zusammensetzung (B) besteht, die in einem kosmetisch annehmbaren Milieu Periodsäure oder dessen wasserlösliche Salze enthält.

**18.** Set mit mehreren Behältern oder Färbekits, dadurch gekennzeichnet, daß sie in einem ersten Behälter eine Zusammensetzung (A) enthalten, wie sie in einem der Ansprüche 1 bis 13, 15 und 16 definiert ist, und in einem zweiten Behälter eine Zusammensetzung (B) enthalten, wie sie in einem der Ansprüche 1 bis 10, 12, 14, 15 und 16 definiert ist.

## Claims

**1.** Process for dyeing keratinous fibres, characterised in that there is applied to these fibres,

in a first step, a composition (A) containing, in a medium suitable for dyeing:

(i) at least one monohydroxyindole corresponding to the formula (I):

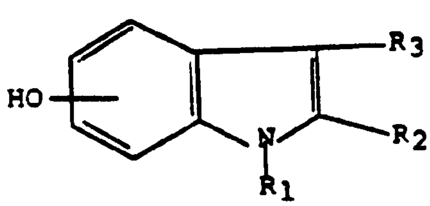

(I)

in which:

$R_1$ denotes hydrogen or a $C_1$-$C_4$ alkyl radical;

$R_2$ and $R_3$ denote, independently of each other, hydrogen or an $NH_2$, OH, COOH, $CH_2COOH$ or $CONH_2$ group; a $C_1$-$C_4$ alkyl radical, optionally substituted with one or more OH or $NH_2$ groups; an alkoxy radical having 1 to 4 carbon atoms; a group $CONHR_4$ or a group $CON(R_5)(R_6)$ or a group $CO_2R_4$, in which $R_4$, $R_5$ and $R_5$ denote an alkyl radical containing 1 to 4 carbon atoms; a halogen atom, and

(ii) at least one 5,6-disubstituted hydroxyindole of formula (II),

or mixture(s) thereof;

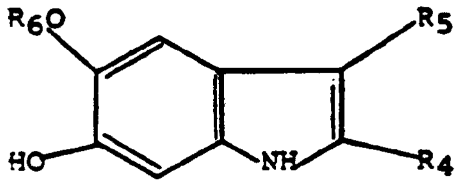

(II)

in which:

$R_6$ denotes hydrogen or acetyl;

$R_4$ denotes hydrogen, methyl, carboxy or ethoxycarbonyl;

$R_5$ denotes hydrogen or methyl,

with the proviso that when $R_6$ denotes acetyl, $R_4$ and $R_5$ represent a hydrogen atom; and its salts;

- in that, in a second step, a rinsing is carried out of the fibres treated with the composition applied in the first step; and
- in that, in a third step, a composition (B) is applied which contains, in an aqueous medium suitable for dyeing, periodic acid or one of its salts which is soluble in this medium.

2. Process according to Claim 1, characterised in that the monohydroxyindole is chosen from 4-hydroxyindole, 5-hydroxyindole, 6-hydroxyindole, 7-hydroxyindole, 5-hydroxy-2-indolecarboxylic acid, 5-hydroxy-3-indoleacetic acid, 5-hydroxy-3-hydroxyethylindole and 5-hydroxy-2-hydroxymethylindole.

3. Process according to Claim 1 or 2, characterised in that the 5,6-disubstituted hydroxyindole of formula (II) is 5,6-dihydroxyindole.

4. Process according to Claim 1 or 2, characterised in that the 5,6-disubstituted hydroxyindole is chosen from 5,6-dihydroxy-2-carboxyindole, 5,6-dihydroxy-2-ethoxycarbonylindole, 5-acetoxy-6-hydroxyindole, 2,3-dimethyl-5,6-dihydroxyindole and 2-methyl-5,6-dihydroxyindole.

5. Process for dyeing keratinous fibres, characterised in that there is applied to these fibres,
   - in a first step, a composition (A) containing, in a medium suitable for dyeing:
     (i) at least one monohydroxyindole chosen from 4-hydroxyindole, 5-hydroxyindole, 6-hydroxyindole and 7-hydroxyindole,
     (ii) at least 5,6-dihydroxyindole;
   - in that, in a second step, the fibres thus treated are rinsed;
   - in that there is applied, in a third step, a composition (B) which contains, in an aqueous medium suitable for dyeing, periodic acid or one of its salts which is soluble in this medium.

6. Process according to any one of Claims 1 to 5, characterised in that the salts of periodic acid are chosen from the salts of lithium, sodium, potassium, rubidium, caesium, magnesium, calcium, strontium, manganese, iron, copper, zinc and aluminium.

7. Process according to any one of Claims 1 to 6, characterised in that the monohydroxyindole of formula (I) and the 5,6-disubstituted hydroxyindole of formula (II) are each present in respective concentrations of between 0.01 and 5 % by weight, preferably between 0.05 and 3 % by weight, with respect to the total weight of the composition.

8. Process according to any one of Claims 1 to 6, characterised in that the periodic acid or its salts are present in proportions of between 0.004 and 0.07 mol and preferably between 0.01 and 0.04 mol per 100 g of composition.

9. Process according to any one of Claims 1 to 8, characterised in that the composition (A) is applied for an

exposure time of 1 minute to 60 minutes before proceeding with the rinsing and the composition (B) is applied with an exposure time of between 1 minute and 60 minutes, it being possible for the exposure times of the compositions (A) and (B) to be identical or different.

**10.** Process according to any one of Claims 1 to 9, characterised in that the compositions used contain an aqueous medium consisting of water or a water/solvent(s) mixture.

**11.** Process according to any one of Claims 1 to 9, characterised in that the medium of the composition (A) consists of an anhydrous solvent medium.

**12.** Process according to any one of Claims 10 or 11, characterised in that the solvents are chosen from aliphatic alcohols, polyols, monomethyl, monoethyl or monobutyl ethers of ethylene glycol, diethylene glycol, propylene glycol or dipropylene glycol, the acetate of the monomethyl ether of ethylene glycol, or methyl lactate.

**13.** Process according to any one of Claims 1 to 10 and 12, characterised in that the composition (A) is aqueous and has a pH of between 3 and 10 and preferably of between 4 and 7.

**14.** Process according to any one of Claims 1 to 10 and 12, characterised in that the composition (B) has a pH of between 2 and 10 and preferably between 2.5 and 5.

**15.** Process according to any one of Claims 1 to 14, characterised in that the medium of the compositions (A) and (B) is thickened with thickening agents present in proportions of between 0.1 and 5 % by weight with respect to the total weight of the composition.

**16.** Process according to any one of Claims 1 to 15, characterised in that the medium of the compositions (A) and (B) also contains the adjuvants normally employed for the dyeing of keratinous fibres, chosen from surface-active agents, sequestering agents, antioxidising or reducing agents, film-forming agents, pearlescence agents, dispersing agents, treating agents, penetrating agents, fragrances, buffers and electrolytes.

**17.** Composition containing several separate components, characterised in that it comprises a first component consisting of a composition (A) containing, in a cosmetically acceptable medium, at least one monohydroxyindole corresponding to the formula (I):

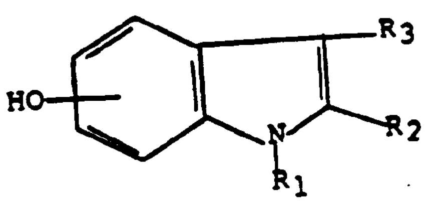

(I)

in which:

$R_1$ denotes hydrogen or an alkyl radical having 1 to 4 carbon atoms;

$R_2$ and $R_3$ denote, independently of each other, hydrogen; an $NH_2$, OH, COOH, $CH_2COOH$, $CONH_2$ group; an alkyl radical containing 1 to 4 carbon atoms, optionally substituted with one or more OH or $NH_2$ groups; an alkoxy radical containing 1 to 4 carbon atoms; a group $CONHR_4$ or a group CON ($R_5$) ($R_6$) or a group $CO_2R_4$, in which $R_4$, $R_5$ and $R_6$ denote an alkyl radical containing 1 to 4 carbon atoms; a halogen atom and at least one 5,6-disubstituted hydroxyindole of formula (II):

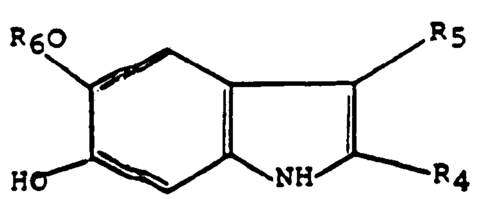

(II)

in which:

$R_6$ denotes hydrogen or acetyl;

$R_4$ denotes hydrogen, methyl, carboxy or ethoxycarbonyl;

$R_5$ denotes hydrogen or methyl,

with the proviso that when $R_6$ denotes acetyl, $R_4$ and $R_5$ represent a hydrogen atom; and its salts;

and a second component consisting of a composition (B) containing, in a cosmetically acceptable medium, periodic acid or its water-soluble salts.

**18.** Device containing several dyeing compartments or kits, characterised in that it comprises a first compartment containing the composition (A) as defined in any one of Claims 1 to 13, 15 and 16 and a second compartment containing the composition (B) as defined in any one of Claims 1 to 10, 12, 14, 15 and 16.